# EUROPEAN PATENT SPECIFICATION

(11) **EP 2 265 625 B1**
(45) Date of publication and mention of the grant of the patent: **12.11.2014**
(21) Application number: 08873364.7
(22) Date of filing: 17.03.2008
(51) Int. Cl.: C07H 3/02, C07H 1/08, C07G 1/00, C07G 3/00

(54) **METHOD OF CONTINUOUS ACID HYDROLYSIS OF CELLULOSE CONTAINING SUBSTANCES**
VERFAHREN ZUR KONTINUIERLICHEN SÄUREHYDROLYSE VON CELLULOSEHALTIGEN SUBSTANZEN
PROCÉDÉ D'HYDROLYSE ACIDE EN CONTINU DE SUBSTANCES CONTENANT DE LA CELLULOSE

(43) Date of publication of application: 29.12.2010
(73) Proprietor: Bio Tech Ltd, Shumen 9700 (BG)
(72) Inventor: CHERNYAVSKAYA, Nina Andreevna, Moscow 117574 (RU)
(74) Representative: Maiwald, Walter
(86) International application number: PCT/RU2008/000148
(87) International publication number: WO 2009/116885

(56) References cited:
- WO-A1-84/03304
- GB-A- 2 003 478
- GB-A- 2 036 826
- RU-A- 2001 130 449
- RU-C2- 2 189 996
- SU-A1- 273 647
- US-A- 3 970 712
- US-A- 4 427 584
- NIKITIN N.I.: 'Khimiya drevesiny i cellyulozy.' IZDATELSTVO AKADEMII NAUK SSSR. 1962, MOSKVA - LENINGRAD, page 434
- SHORYGINA N.N. ET AL.: 'Reakcionnaya sposobnost Lignina.' NAUKA 1976, MOSKVA, page 335

## Description

### PERTINENT ART

This invention relates to a method of producing monosaccharides by means of acid hydrolysis of cellulose containing substances.

Hydrolysis production methods are used for manufacturing food products, fodder products and technical products from non-edible plant raw materials: logging waste, sawmill and wood processing waste (wood meal, fine chips, chipped wood, bark, etc.), agricultural processing waste (straw, seeds husk, sunflower seeds peel, com cobs and stalks, shive, cane, fast-growing grass like sugar sorghum, etc.), household and communal services wastes, i.e. any material containing cellulose and hemicellulose in any form.

### BRIEF DESCRIPTION OF THE PRIOR ART

The hydrolysis production method is based on hydrolysis of raw materials (transformation of polysaccharides contained therein to monosaccharides) that undergo further chemical and biochemical processing.

The known processes of cellulose hydrolysis include biological and non-biological methods of depolymerizing. Biological methods provide for cellulose hydrolysis under the effect of ferments (enzymes). Among non-biological methods, the traditional and the most well known (applied when manufacturing sugar from cellulose) is the method of hydrolysis in an acid medium. Sulfuric acid is used most frequently in this method - in most case diluted.

Sulfuric acid with a concentration from 0.5 to 15% is usually used in the process of cellulose hydrolysis. The process of hydrolysis requires a temperature range from 90°C to 300°C (commonly from 140°C to 240°C) and high pressure. At high temperatures sugars degrade, forming Furfurol as well as other undesirable by- products. As a result, the yield of monosaccharides is very low - usually less than 50%. Therefore, using hydrolysis in a diluted acid medium it is not possible to produce sugars from cellulose containing substances in large quantities and at a competitive price.

Methods of hydrolysis in concentrated sulfuric acid are more efficient and the yield of sugar is higher. In these methods sulfuric acid with a concentration from 60% to 90% is usually used. Despite the fact that these methods were successful, providing a yield of sugar of over 90%, they could not be economically implemented due to the high cost of concentrated sulfuric acid and of its further regeneration, difficulties in handling concentrated sulfuric acid as well as the necessity of equipment which is acid resistant at high temperatures. The process is inherently uneconomical because the greater is the concentration of the applied acid, the more energy is required for its concentration.

Common disadvantages of the known acid hydrolysis methods are: utilization of large equipment; high levels of heat consumption for hydrolysis; time consuming process (several hours) due to a low rate of hydrolysis; low delignification of the reaction products resulting in the contamination of hydrolysates (monosaccharides solutions) with by-products - considerably lowering their quality.

The disadvantage of the known methods is a low yield of monosaccharides due to degradation in the reaction zone while exposed to high temperatures and pressure, and the separation of Furfurol and other by-products.

Attempts at overcoming this disadvantage (Patent No. SU1701115 (1982) as well as its updated version WO84/03304) by means of adding acetone into the hydrolysis process create compounds with monosaccharides that increase monosaccharides resistance to high temperatures. The above said documents contain a description of the process of producing sugars from a cellulose containing substance by a method of autoclave hydrolysis in diluted acid with the implementation of a digestion liquor containing 0.001-0.1 normal sulfuric or hydrochloric acid, water and acetone (with a concentration of acetone from 70% to 90%) at temperatures from 145°C to 230°C with five consecutive treatments of the same amount of initial material.

The main disadvantages of the known method include the use of a two-stage process for separating monosaccharides from cellulose containing substances: in the first stage the initial raw materials hydrolysis takes place at 180°C with a "cooking liquor acetone / water" ratio of 80:20 and 0.04 N H₂SO₄, using 5 treatments of the same sample with the formation (according to the authors' opinion) of diacetone compounds with sugars - these compounds being stable at high temperatures. In the second stage the additional hydrolysis of the obtained reaction products is carried out at a temperature of 100°C during a 40 minute period in the presence of 2.0 N sulfuric acid with the purpose of decomposing the diacetone compounds and separating the monosaccharides. In the first stage of the autoclave hydrolysis, in accordance with SU1701115, there is no provision for the separation of lignin, and additional hydrolysis at the second stage of the process takes place in its presence. Thus, lignin further separated from the reaction products is sulfonated and due to this it appears to be a product of such a process, which can be hardly further utilized. In accordance with the updated method of WO 84/03304 it is suggested that crude lignin should be refined by a repeated dissolution in acetone, filtration from insoluble residual matters with further precipitation into a large excess of water (circulation of large volumes of water in the processing procedure), or by concentrating the acetone solution. In WO 84/03304 it is suggested that bicarbonates should be used for fast neutralization of solutions containing diacetone compounds, however, such a method of neutralization will lead to emissions of large volumes of carbon dioxide.

The use of 70-90% acetone (acetone/water ratio is 9:1; 4:1; 2.33:1) in a digestion liquor is also a disadvantage of this process as this requires large volumes of acetone to be recycled.

GB 2 003 478 A describes a multi-stage process for the production of sugars from lignocellullosic raw materials which comprises subjecting the raw materials tio a chemical pretreatment with a mixture of water and lower aliphatic alcohols and/or ketones at a temperature from 100 to 190°C for a period of from 4 hours to 2 minutes, followed by a separation of residue and a subsequent main chemical treatment with a similar solvent mixture at elevated temperatures for a further period of from 6 hours to 2 minutes. After removing fibrous materials, the soluble oligosaccharides and polysaccharides are hydrolysed by addition of an acid, with separation of the organic solvent and lignin.

### OBJECTS AND SUMMARY OF INVENTION

The objective of this invention is to provide a method of acid hydrolysis of cellulose containing substances, which protects the monosaccharides from high temperatures and pressure thus increasing their yield, avoids the disadvantages of the known method, and at the same time provides the following:
- simplification of the process by carrying out the hydrolysis in one stage;
- obtaining pure unsolfonated lignin along with monosaccharides at the output;
- removing the necessity to recycle large volumes of a monosaccharide protector.

The said tasks, as well as a number of others are accomplished by means of this invention by acid hydrolysis of cellulose containing substances, comprised of the following steps:
- loading initial feed, if necessary preliminary disintegrated, into a reaction chamber;
- supplying hydrolyzing fluid in the form of a diluted acid at pH<5 into a reaction chamber provided by means of mineral or organic acids, salts and Lewis acids;
- carrying out the acid hydrolysis reaction at temperatures of 140°C - 240°C under increased pressure with monosaccharide protectors that protect them from the impact of high temperatures and pressure, that are able to break hydrogen bonds between macromolecules of cellulose, and to create compounds with monosaccharides by means of hydrogen bonds;
- withdrawal of the reaction products from the reaction chamber;
- separation of lignin from the reaction products;
- separation of monosaccharides from protectors; a single stage hydrolysis is being carried out;
- separation of lignin from the reaction products is carried out either before or after separation of monosaccharides from protectors by its precipitation with further filtration, and
- separation of monosaccharides from protectors is carried out by distilling the reaction products without additional hydrolysis.

The monosaccharides protectors are methyl-ethyl ketone and/or 1.4-dioxane.

A mixture of protectors will considerably reduce the pressure in the reaction chamber, which provides for a more secure process as well as simpler equipment. The ratio of the protector or protectors to water in the hydrolyzing fluid might be substantially lower than provided by the SU1701115 method - going from 2:1 to 1:2.

In accordance with the invention, the withdrawal of the reaction products is carried out by means of their discharge from the reaction chamber to expansion tanks through a pressure reducing throttling device; in such a case, rapid cooling of the reaction products takes place along with separation of volatile reaction products (particularly furfurol and its derivatives).

At higher lignin concentration in feed, part of it can be withdrawn from the initial feed by means of alkaline pulping prior to loading into the reaction chamber. Along with the generally accepted types of raw materials, it is possible to use a primary sedimentary slush from sewage disposal plants for sewage and/or household wastes.

The above said characteristics and advantages of the invention will become more clear from its detailed description below, which is given in the form of the invention's embodiment examples, not of a restrictive character, and is provided with reference to the accompanying drawings.

### BRIEF DESCRIPTION OF THE DRAWINGS

Fig. 1 represents an option of a process flow sheet of the method's embodiment in accordance with the invention.
Fig. 2 represents another option of a process flow sheet of the method's embodiment in accordance with the invention.

### DETAILED DESCRIPTION OF THE INVENTION

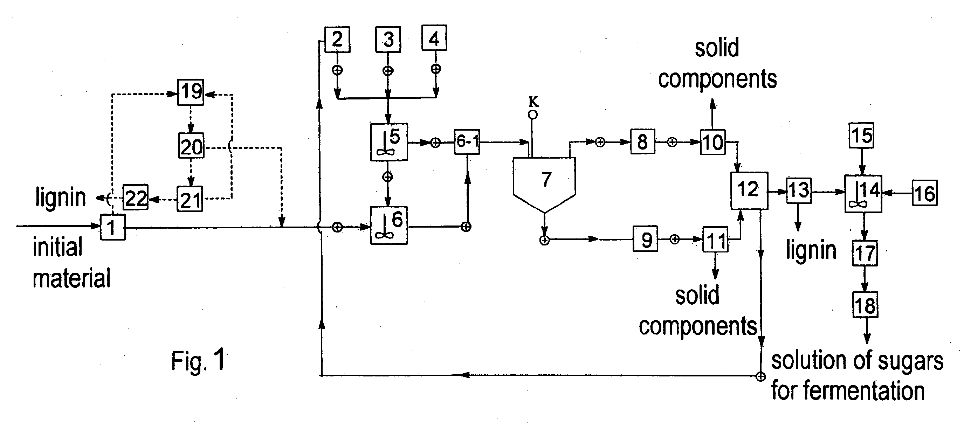

Let us review Fig. 1 which represents the process flow sheet of the method's application when a separation of monosaccharides from the protector (protectors) is carried out prior to a separation of lignin from the reaction products.

The initial material is supplied to a "blender" - Batcher 1 (or without preliminary disintegration as, for example, in the case of using a primary sedimentary slush from sewage disposal plants). From Batcher 1 a solid material with particles of 0.5-1.0 mm is supplied to Mixing Tank 6. Batch meters - Batchers 2, 3, 4 contain liquid components of the reaction mixture that are supplied to Mixing Tanks 5, 6. Reactor 7 is equipped with, not shown on the Diagram, heating jacket, temperature-sensing, pressure-sensing devices, safety valve, siphon pipe connected with the upper valve (throttle) for the reaction products discharge, lower valve (throttle) for the reaction products discharge, (as well with a faucet for steam supply directly into Device 7, as may be required, for quickly heating the reaction mass and supplying part of the required water quantity for hydrolyzing solution or for pre-steaming the initial raw materials prior to their treatment with hydrolyzing solutions, if it is required). From Mixing Tanks 6 and 5 the reaction mixture either with the initial solid material or without it is supplied to Reactor 7 through the pump - Batcher 6 - 1 and through the back-flow valve "K". The reaction products are discharged from Reactor 7 through the valves (throttles) of Device 7 into Expansion Tanks 8 and 9, where they rapidly cool and separate from volatile by-products that are, in their turn, discharged through the valves from Expansion Tanks 8 and 9 into collecting facilities (not shown). After that, through Filters 10 and 11, where solid components of the reaction mass are separated, the reaction products' solution is supplied to Distillation Apparatus 12, where protectors (or at least one of them) are separated and then returned to batchbox - Batcher 2. The residual mass is supplied to Filter (or hydrocyclone) 13, where the lignin is separated, and then proceeds to "Neutralizer" 14, which is equipped with a pH meter (15), batchbox - Batcher (16). Neutralized products (water solution of sugars) pass through Filter 17, are collected in Receiver Tank 18 and are forwarded for fermentation.

With regard to some cellulose containing materials with rather high volume of lignin a preliminary treatment of these raw materials can be provided, for example, by means of "alkaline pulping" in a diluted alkaline solution such as a 4% water solution of NaOH. In this case, a preliminary treatment block is added to the process flow sheet (Diagram 1, items No. 19 - 22). The disintegrated product is supplied through Batcher 1 to "Boiling Tank" 19, where it is treated with an alkaline solution (T= 95°C - 100°C; 2 hours) and boiling extraction of the majority of the lignin contained in the initial substrate takes place. Then through Filter 20, where alkali cleaning from a solid substrate takes place, it joins the processing chain of Diagram 1 through Item 6 and the alkaline solution of lignin is forwarded to Catcher 21, where lignin is swaged. Alkali is returned back to the processing cycle and the sediment of lignin is withdrawn from the process through Centrifuge (hydrocyclone) 22.

The procedure can carry out multiple treatments of the initial material with new portions of the hydrolyzing fluid. In such a case the reactor is first supplied through a dosing pump with the reaction mixture along with cellulose containing substance sample from Mixing Tank 6, and then after holding, the liquid reaction products are discharged through the upper valve (throttle) into the Expansion Tank. In this case the treated solid sample is left in the reaction chamber. Fresh hydrolyzing fluid is supplied from Mixing Tank 5 by a dosing pump, which is switched (possibly automatically) to this mixing tank. For 1 intake with the sample from Mixing Tank 6 there are 2 intakes of pure hydrolyzing solution from Mixing Tank 5. The valves for the products' "discharge" also function in turns: for 2 discharges through the upper valve (throttle) there is 1 discharge through the lower valve (throttle), and in this case the third treatment of the material sample's residual mass takes place.

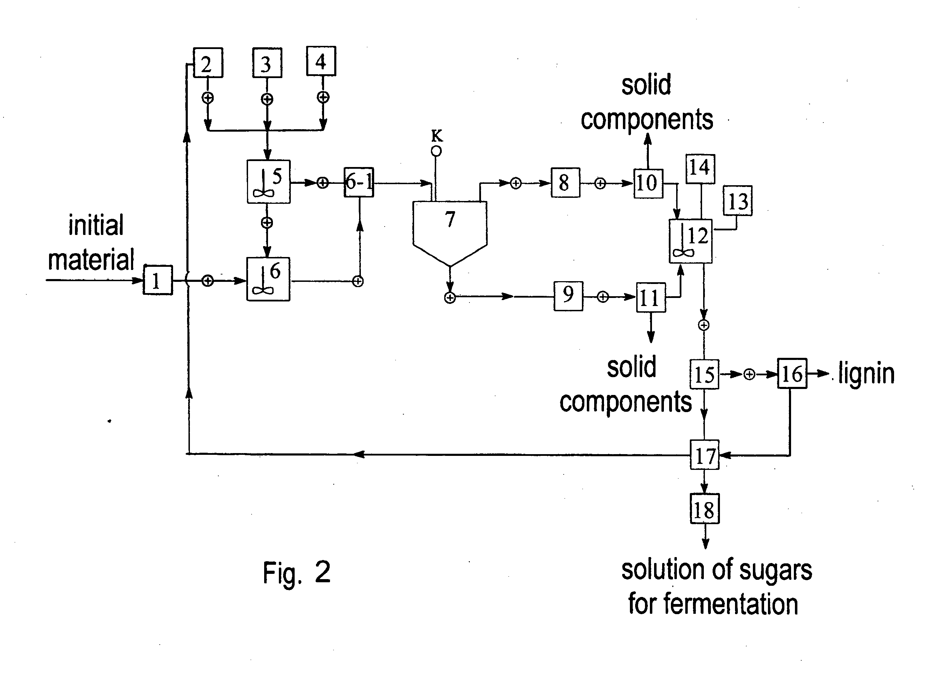

Let us now review Fig. 2 which represents the process flow sheet of the method's embodiment when a separation of monosaccharides from the protector is carried out after a separation of lignin from the reaction products.

The initial material is supplied to a "blender" - Batcher 1 (or without preliminary disintegration as, for example, in the case of using a primary sedimentary slush from sewage disposal plants). From Batcher 1, a solid material with particles of 0.5-1.0 mm is supplied to Mixing Tank 6. Batch meters - Batchers 2, 3, 4 contain liquid components of the reaction mixture that are supplied to Mixing Tanks 5, 6. From Mixing Tanks 6 and 5 the reaction mixture, either with the initial solid material or without it, is supplied to Reactor 7 through the pump - Batcher 6 - 1 and through the back-flow valve "K". The reaction products are discharged from Reactor 7 through the valves (throttles) of Device 7 into Expansion Tanks 8 and 9, where they quickly cool and separate from volatile by-products that are, in their turn, discharged through the valves from Expansion Tanks 8 and 9 into collecting facilities. After that, thorough Filters 10 and 11, where solid components of the reaction mass are separated, the reaction product solution is supplied to "Titrator" 12 equipped with a pH meter (14), batch meter - batcher of the titrating solution (13), where pH is increased to 10. Then this product is supplied into Catcher 15, where the lignin is separated and precipitates out of solution, then is separated through Filter (hydrocyclone) 16, and the upper layer of the product after neutralization is forwarded from 15 to "Distillation Apparatus" 17, where the protector (or the protectors' mixture) is separated and is returned to batch meter - Batcher 2. The residual mass (water solution of sugars) is collected through a filter (hydrocyclone) in Receiver Tank 18 and is forwarded for fermentation.

The following are examples of the embodiment of the invention with the use of different input substances and with different monosaccharide protectors.

### Comparative Example 1

130 g of a primary sedimentary slush from municipal sewage disposal plants with 30% humidity (100 g of the dry initial material) was loaded into the reaction chamber. It was treated 3 times of 5 minutes each at T=180°C and pH = 1.87 with hydrolyzing solution (HS) at the "initial material / hydrolyzing solution" ratio of 1:10 and at the "protector / water in hydrolyzing solution" ratio of 4:1 (protector - acetone). HS was loaded through the back-flow valve by the dosing pump and the reaction products were discharged through the upper and lower autoclave throttles into the expansion tanks as described above (Diagram 1).

After its filtration and separation the unreacted primary slush was dried to its permanent weight (32 g). After filtration the protector was withdrawn from the merged hydrolyzing solutions by distilling it in the vacuum of the water-jet pump. The lignin sediment was separated by means of filtration. The lignin sediment on the walls of the splitter flask was flushed away and dried. Cumulative yield of lignin amounted to 28.6 g. The residual mass (water solution of sugars) was neutralized to the level of pH = 6.1 by "calcium milk" (CaO), and the precipitation was filtrated. The concentration of sugars in the water solution was defined by means of a saccharimeter. The yield of sugars amounted to 32.5 g. The volatile reaction products were gathered in the storage flask through the faucets of the radiator feed tanks. Their approximate content - 6.9 g (Table 1, Paragraph 1).

### Example 2

The triple autoclave treatment was applied to 146 g of bagasse with 46% humidity of the initial sample (100 g of the dry sample) at pH = 2.5; at a temperature of 180°C; duration of each treatment was 5 minutes. The "HS / sample and protector / water" ratio was similar to the one in Example 1. The protector was a mixture of methyl-ethyl ketone with acetone at a 1:1 ratio. The treatment method was similar to the one in Example 1. The residual mass of the sample amounted to 18 g. The yield of sugars and lignin amounted to 62 g and 18 g respectively (Table 1, Paragraph 2).

### Example 3

The triple autoclave treatment was applied to 146 g of bagasse with 46% humidity of the initial sample (100 g of the dry sample) at pH = 3.0; at a temperature of 210°C; duration of each treatment was 3 minutes. The HS / sample and protector / water ratio was similar to the one in Example 1. The protector was methyl-ethyl ketone. The treatment method was similar to the one in Example 1. The residual mass of the sample amounted to 9 g. The yield of sugars and lignin amounted to 65 g and 20 g respectively (Table 1, Paragraph 3).

### Example 4

A straw sample of 110 g with 4.82% humidity was placed into the autoclave and treated three times with a duration of 4 minutes each at T = 180 °C; pH = 1.6. The "HS / sample and protector / water" ratio was similar to the one in Example 1. The protector was methyl-ethyl ketone. The treatment method was similar to the one in Example 1. The residual mass of the sample amounted to 18.2 g. The yield of sugars and lignin amounted to 62.66 g and 20.2 g respectively (Table 1, Paragraph 4).

### Example 5

A straw sample received a preliminary treatment with a 4% solution of NaOH at a temperature of 97°C - 100°C for two hours ("alkaline pulping"). After that, the sample was filtrated from the solution, flushed out at a filter from the remains of the alkali solution and dried. In the course of such a treatment the majority of the lignin is released from the straw sample (the residual matter of lignin in the straw may amount to about 11%) along with part of the hemicellulose. The remains of the straw from the initial sample taken for the treatment amounts to 41.7% on average. If necessary, some quantities of pentose sugars can be extracted from the filtrates.

The treated straw sample (as described above) with a weight of 110 g and with 10% humidity was placed into the autoclave, where it was treated three times (4 minutes each) at T = 180°C; pH = 2.1; HS at the protector / water ratio of 1:1 (protector was methyl-ethyl ketone), the "sample / HS" ratio of 1 : 10. The merged hydrolysates were placed into the "titrator" (as was described above, Diagram 2), pH value was brought up to 10 and the solution was allowed to settle so that lignin sediment could form.

Lignin was separated by filtration and dried (10 g). After the neutralization, methyl-ethyl ketone was withdrawn from the filtrate in the vacuum of the water-jet pump. The yield of sugars amounted to 64.2 g. The residual matter of the sample after the hydrolysis amounted to 17.68 g (Table 1, Paragraph 5).

### Example 6

The straw sample pretreated in the same way as in Example 5, of 99 g in terms of a dry straw, was treated three times (10 minutes each) in the autoclave at T = 150°C; pH = 4.5, with the solution, which contained a mixture of 1.4-dioxane and water at the ratio of 1 : 2.12 correspondingly. The "sample / hydrolyzing solution" ratio was similar to the one in the previous experiments. The separation of the reaction products was carried out in accordance with the methods used in Experiment 1. The yield of sugars amounted to 57.6 g. The residual matter of the sample after treatment amounted to 17.2 g. The yield of lignin amounted to 4.15 g (Table 1, Paragraph 6).

### Comparative Example 7

50 g of coniferous wood meal (in terms of a dry sample) was treated in the autoclave three times (6 minutes each) at T = 190°C; pH = 1.87, with a solution that contained a mixture of acetone and water at the ratio of 4 : 1 at the same level of dilution as in the previous experiments. The separation of the reaction products was carried out in accordance with the methods used in Experiment 1.

The yield of sugars amounted to 35.6 g; that of lignin - to 30 g. Conversion of the initial sample reached 71.6% (Table 1, Paragraph 7).

**Table 1**

| No. | Type of material (dry shot), g | Conversion (in terms of a dry material), % | Yield of sugars, % | Yield of lignin, % | Yield of by-products, % |
|---|---|---|---|---|---|
| 1 | Primary sedimentary slush (100) | 68 | 32.5 | 28.6 | 6.9 |
| 2 | Bagasse (100) | 82 | 62 | 18 | 2.0 |
| 3 | Bagasse (100) | 90.2 | 65 | 20 | 5.2 |
| 4 | Straw (104.94) | 82.7 | 59.7 | 19.2 | 3.7 |
| 5 | Straw (99) | 82.1 | 64.9 | 10 | 7.2 |
| 6 | Straw (99) | 65.3 | 58.2 | 4.2 | 21.2 |
| 7 | Wood meal of coniferous trees (100) | 71.6 | 35.6 | 30 | 6.0 |

### Industrial applicability

As was established, the method in accordance with this invention enables the hydrolysis of cellulose containing substances that range from timber waste and bagasse to primary sedimentary slush from sewage disposal plants.

The high yield of sugars and the high quality of the separated lignin is inherent in the process; the reaction of hydrolysis takes place in one stage (in one reaction chamber) during a short period of time, which ensures the cost effectiveness of the process.

## Claims

1. A method of acid hydrolysis of a cellulose containing material, comprising the steps of:
- loading initial raw materials, if necessary preliminary disintegrated, into a reaction chamber;
- supplying hydrolyzing fluid in the form of a diluted acid at pH<5 into a reaction chamber;
- carrying out the acid hydrolysis reaction at temperatures of 140°C - 240°C under increased pressure with the presence of methyl-ethyl-ketone and/or 1,4-dioxane;
- withdrawal of the reaction products from the reaction chamber;
- separation of lignin from the reaction products;
- separation of monosaccharides from the methyl-ethyl-ketone and/or 1,4-dioxane; wherein hydrolysis is provided in a single stage, and wherein separation of monosaccharides from methyl-ethyl-ketone and/or 1,4-dioxane is carried out by distilling the reaction products without additional hydrolysis, and wherein separation of lignin from the reaction products is carried out either before or after separation of monosaccharides from the methyl-ethyl-ketone and/or 1,4-dioxane by its precipitation with further filtration.

2. The method of Claim 1 wherein a mixture of methyl-ethyl-ketone and 1,4-dioxane is used.

3. The method of any one of above Claims wherein the ratio of methyl-ethyl-ketone and/or 1,4-dioxane to water in the hydrolyzing fluid is from 2:1 to 1:2.

4. The method of Claim 1 wherein the discharge of the reaction products is carried out by means of dumping the contents of the reaction chamber into the expansion tanks through a pressure decreasing throttling device.

5. The method of any one of above Claims wherein the separation of lignin from the reaction products is carried out prior to the separation of monosaccharides from the methyl-ethyl-ketone and/or 1,4-dioxane at pH = 10.

6. The method of any one of Claims 1 to 4 wherein the separation of lignin from the reaction products is carried out after the separation of monosaccharides from the methyl-ethyl-ketone and/or 1,4-dioxane.

7. The method of Claim 1 wherein with a higher lignin concentration in raw materials, a part of it is withdrawn from the initial raw materials by means of an alkaline pulping prior to loading into the reaction chamber.

8. The method of Claim 1 wherein a primary sedimentary slush from sewage disposal plants for sewage and/or household wastes is used as an initial raw material.

## Patentansprüche

1. Ein Verfahren zur sauren Hydrolyse eines Zellulose-enthaltenden Materials, umfassend die folgenden Schritte:
- Befüllen einer Reaktionskammer mit ursprünglichen Rohmaterialien, die falls notwendig bereits teilweise zersetzt sind;
- Zugabe von hydrolysierender Flüssigkeit in Form einer verdünnten Säure mit pH<5 in die Reaktionskammer;
- Durchführen der sauren Hydrolysereaktion bei Temperaturen von 140°C - 240°C bei erhöhtem Druck in Anwese eit von Methylethylketon und/oder 1,4-dioxan,
- Abziehen der Reaktionsprodukte aus der Reaktionskammer;
- Trennung von Lignin von den Reaktionsprodukten;
- Trennung von Monosacchariden vom Methylethylketon und/oder 1,4- dioxan; wobei die Hydrolyse in einem Schritt durchgeführt wird, und wobei die Trennung der Monosaccharide vom Methylethylketon und/oder 1,4-Dioxan ausgeführt wird, indem die Reaktionsprodukte ohne zusätzliche Hydrolyse destilliert werden, und wobei die Trennung des Lignin von den Reaktionsprodukten entweder vor oder nach der Trennung der Monosaccharide vom Methylethylketon und/oder 1,4-Dioxan durch Fällung und anschließende Filtration ausgeführt wird.

2. Das Verfahren gemäß Anspruch 1, wobei eine Mischung aus Methylethylketon und 1,4-Dioxan verwendet wird.

3. Das Verfahren gemäß irgendeinem der vorhergehenden Ansprüche, wobei das Verhältnis von Methylethylketon und/oder 1,4-Dioxan zu Wasser in der hydrolysierenden Flüssigkeit von 2:1 bis 1:2 beträgt.

4. Das Verfahren gemäß Anspruch 1, wobei das Abziehen der Reaktionsprodukte ausgeführt wird mittels Abgabe des Inhalts der Reaktionskammer in die Expansionstanks durch eine drucksenkende Drosselvorrichtung.

5. Das Verfahren gemäß irgendeinem der vorhergehenden Ansprüche, wobei die Trennung des Lignins von den Reaktionsprodukten ausgeführt wird vor der Trennung der Monosaccharide vom Methylethylketon und/oder 1,4-Dioxan, bei pH = 10.

6. Das Verfahren gemäß irgendeinem der Ansprüche 1 bis 4, wobei die Trennung des Lignins von den Reaktionsprodukten ausgeführt wird nach der Trennung der Monosaccharide vom Methylethylketon und/oder 1,4-Dioxan.

7. Das Verfahren gemäß Anspruch 1, wobei bei einer höheren Ligninkonzentration in den Rohmaterialien ein Teil davon aus den ursprünglichen Rohmaterialien entfernt wird mittels eines alkalischen Auslaugens vor dem Befüllen der Reaktionskammer.

8. Das Verfahren gemäß Anspruch 1, wobei ein primärer Sedimentschlamm aus Abwasserentsorgungsanlagen für Abwasser und/oder Haushaltsmüll als ein ursprüngliches Rohmaterial verwendet wird.

## Revendications

1. Procédé d'hydrolyse acide d'une matière contenant de la cellulose, comprenant les étapes qui consistent à :
- charger les matières premières initiales, préalablement désintégrées le cas échéant, dans une chambre de réaction ;
- fournir du liquide hydrolysant sous la forme d'un acide dilué à pH < 5 dans une chambre de réaction ;
- effectuer la réaction de l'hydrolyse acide à des températures de 140 °C à 240 °C sous une pression accrue en présence de méthyl-éthyl-cétone et/ou de 1,4-dioxane ;
- retirer les produits de réaction de la chambre de réaction ;
- séparer la lignine des produits de réaction ;
- séparer les monosaccharides du méthyl-éthyl-cétone et/ou du 1,4-dioxane ;
dans lequel l'hydrolyse est obtenue en une seule étape, et dans lequel les monosaccharides sont séparés du méthyl-éthyl-cétone et/ou du 1,4-dioxane en distillant les produits de réaction sans hydrolyse supplémentaire, et dans lequel la lignine est séparée des produits de réaction avant ou après avoir séparé les monosaccharides du méthyl-éthyl-cétone et/ou du 1,4-dioxane en la précipitant et en effectuant une filtration supplémentaire.

2. Procédé selon la revendication 1, dans lequel un mélange de méthyl-éthyl-cétone et de 1,4 dioxane est utilisé.

3. Procédé selon l'une quelconque des revendications précédentes, dans lequel le rapport entre le méthyl-éthyl-cétone et/ou le 1,4-dioxane et l'eau dans le liquide hydrolysant est de 2:1 à 1:2.

4. Procédé selon la revendication 1, dans lequel les produits de réaction sont évacués en déversant le contenu de la chambre de réaction dans les réservoirs d'expansion par l'intermédiaire d'un dispositif d'étranglement réducteur de pression.

5. Procédé selon l'une quelconque des revendications précédentes, dans lequel la lignine est séparée des produits de réaction avant d'avoir séparé les monosaccharides du méthyl-éthyl-cétone et/ou du 1,4-dioxane à pH = 10.

6. Procédé selon l'une quelconque des revendications 1 à 4, dans lequel la lignine est séparée des produits de réaction après avoir séparé les monosaccharides du méthyl-éthyl-cétone et/ou du 1,4-dioxane.

7. Procédé selon la revendication 1, dans lequel avec une concentration de lignine plus élevée dans les matières premières, une portion de cette dernière est retirée des matières premières initiales par l'intermédiaire d'un pulpage alcalin avant le chargement dans la chambre de réaction.

8. Procédé selon la revendication 1, dans lequel un lisier sédimentaire primaire issu de stations d'épuration d'eaux usées et/ou de déchets domestiques fait office de matière première initiale.
